(19) Europäisches Patentamt
European Patent Office
Office européen des brevets

(11) Numéro de publication : **0 606 182 A1**

(12) # DEMANDE DE BREVET EUROPEEN

(21) Numéro de dépôt : **94400019.9**

(51) Int. Cl.$^5$ : **C07C 37/60,** C07C 39/08

(22) Date de dépôt : **05.01.94**

(30) Priorité : **08.01.93 FR 9300121**

(43) Date de publication de la demande : **13.07.94 Bulletin 94/28**

(84) Etats contractants désignés : **DE FR GB IT NL SE**

(71) Demandeur : **RHONE-POULENC CHIMIE
25, quai Paul Doumer
F-92408 Courbevoie Cédex (FR)**

(72) Inventeur : **Costantini, Michel
10, rue du Docteur Bonhomme
F-69003 Lyon (FR)**
Inventeur : **Manaut, Daniel
2, rue Albert Camus
F-69330 Meyzieu (FR)**
Inventeur : **Michelet, Daniel
6, allée de la Forêt Marly
F-78860 Saint-Nom-la-Breteche (FR)**

(74) Mandataire : **Dutruc-Rosset, Marie-Claude
RHONE-POULENC INTERSERVICES
Service Brevets Chimie
25, Quai Paul Doumer
F-92408 Courbevoie Cédex (FR)**

(54) **Procédé de préparation d'un composé aromatique o-dihydroxylé.**

(57) La présente invention a pour objet un procédé de préparation de composés aromatiques dihydroxylés. L'invention concerne plus particulièrement la préparation de composés aromatiques o-dihydroxylés, et notamment la préparation de la pyrocatéchine.

Le procédé de préparation d'un composé aromatique o-dihydroxylé selon l'invention, est caractérisé par le fait qu'il consiste à faire réagir une o-fuchsone avec un agent oxydant.

EP 0 606 182 A1

La présente invention a pour objet un procédé de préparation de composés aromatiques dihydroxylés. L'invention concerne plus particulièrement la préparation de composés aromatiques o-dihydroxylés, et notamment la préparation de la pyrocatéchine.

De nombreux procédés d'hydroxylation des phénols sont décrits dans l'état de la technique.

Citons, entre autres, les brevets FR-A 2 071 464 et FR-A 2 266 683.

Les procédés connus conduisent à un mélange de diphénols et les quantités d'isomères ortho ou para formées sont sensiblement égales, avec prédominance de l'isomère ortho.

Il s'avère que pour répondre à la demande du marché qui est fluctuante, il est important, de disposer d'un procédé industriel permettant d'atteindre plus sélectivement l'un des isomères.

La demanderesse propose un procédé perfectionné permettant d'obtenir sélectivement des composés aromatiques dihydroxylés en position ortho.

Plus précisément, la présente invention a pour objet un procédé de préparation d'un composé aromatique o-dihydroxylé caractérisé par le fait qu'il consiste à faire réagir un agent oxydant, avec une o-fuchsone.

Dans l'exposé qui suit de la présente invention, on entend par "o-fuchsone", tout composé chimique comportant le motif méthylène-1,2 quinone suivant :

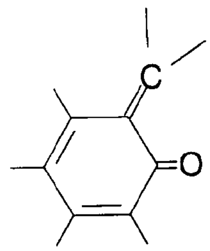

Le produit final est un composé aromatique o-dihydroxylé. Par "composé aromatique", on entend la notion classique d'aromaticité telle que définie dans la littérature, notamment par Jerry MARCH, Advanced Organic Chemistry, 3ème édition, John Wiley and Sons, 1985, p.37 et suivantes.

Conformément au procédé de l'invention, on obtient un composé aromatique o-dihydroxylé, par oxydation d'une o-fuchsone.

L'o-fuchsone mise en oeuvre peut être mise en jeu sous forme anhydre ou hydratée ou en mélange des deux formes.

Ainsi, l'o-fuchsone non hydratée peut être représentée par la formule générale (I) :

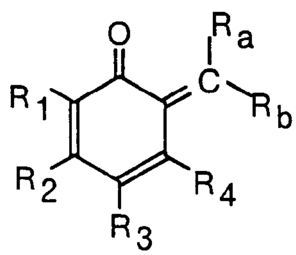

(I)

. dans ladite formule (I), les différents symboles $R_1$, $R_2$, $R_3$, $R_4$, $R_a$ et $R_b$ ont la signification suivante :

- $R_a$ et $R_b$, identiques ou différents sont des radicaux hydrocarbonés ayant chacun de 3 à 30 atomes de carbone ; les atomes de carbone de chaque radical $R_a$ et $R_b$ en position $\alpha$ par rapport à l'atome de carbone qui les porte, étant des carbones tertiaires,
- $R_1$, $R_2$, $R_3$ et $R_4$, identiques ou différents représentent un atome d'hydrogène ou un substituant quelconque,
- deux groupes $R_1$ et $R_2$ et/ou $R_2$ et $R_3$ et/ou $R_3$ et $R_4$ placés sur deux atomes de carbone vicinaux peuvent former ensemble et avec les atomes de carbone qui les portent un cycle.

Il n'y a aucun inconvénient à partir d'une o-fuchsone sous forme hydratée que l'on nomme "carbinol" et qui peut être représentée par la formule suivante (II) :

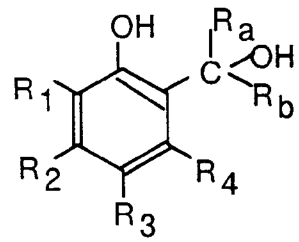

(II)

dans ladite formule (II), les différents symboles $R_1$, $R_2$, $R_3$, $R_4$, $R_a$ et $R_b$ ont les significations données précédemment.

Il est également possible de partir d'un mélange de fuchsones de formule (I) et (II).

Les fuchsones de formule (I) et (II) peuvent être porteuses d'un ou plusieurs substituants $R_1$, $R_2$, $R_3$ ou $R_4$. Des exemples de substituants sont donnés ci-après mais cette liste ne présente aucun caractère limitatif. N'importe quel substituant peut être présent sur le cycle dans la mesure où il n'interfère pas au niveau du produit désiré.

Le procédé de l'invention s'applique plus préférentiellement aux composés de formule (I) et/ou (II), dans laquelle :

- $R_1$, $R_2$, $R_3$ et $R_4$, identiques ou différents représentent $R_0$, l'un des groupes suivants :
  . un atome d'hydrogène,
  . un radical alkyle, linéaire ou ramifié, ayant de 1 à 6 atomes de carbone, de préférence de 1 à 4 atomes de carbone, tel que méthyle, éthyle, propyle, isopropyle, butyle, sec-butyle, tert-butyle,
  . un radical alcényle linéaire ou ramifié ayant de 2 à 6 atomes de carbone, de préférence, de 2 à 4 atomes de carbone, tel que vinyle, allyle,
  . un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy,
  . un groupe acyle ayant de 2 à 6 atomes de carbone,
  . un radical de formule :

$$-R_0-OH$$
$$-R_5-COOR_6$$
$$-R_0-X$$
$$-R_0-CF_3$$

dans lesdites formules, $R_0$ représente un lien valentiel ou un radical hydrocarboné divalent, linéaire ou ramifié, saturé ou insaturé, ayant de 1 à 6 atomes de carbone tel que, par exemple, méthylène, éthylène, propylène, isopropylène, isopropylidène ; $R_6$ représente un atome d'hydrogène ou un radical alkyle linéaire ou ramifié ayant de 1 à 6 atomes de carbone ; X symbolise un atome d'halogène, de préférence un atome de chlore, de brome ou de fluor.

- $R_1$, $R_2$, $R_3$ et $R_4$, identiques ou différents représentent $R_7$, l'un des radicaux plus complexes suivants :
  . un radical carbocyclique saturé ou insaturé ayant de 4 à 7 atomes de carbone, de préférence, un radical cyclohexyle,
  . un radical de formule

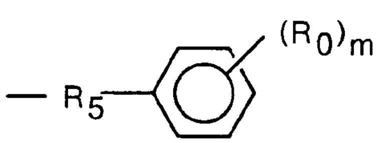

dans lequel $R_5$ représente un lien valentiel ou un radical hydrocarboné divalent, linéaire ou ramifié, saturé ou insaturé ayant de 1 à 6 atomes de carbone tel que par exemple, méthylène, éthylène, propylène, isopropylène, isopropylidène et $R_0$ ayant la signification donnée précédemment et m est un nombre entier de 0 à 4,

  . un radical - $R_5$ - A - $R_0$ dans lequel $R_5$ a la signification donnée précédemment, $R_8$ représente un radical alkyle linéaire ou ramifié ayant de 1 à 6 atomes de carbone ou un radical

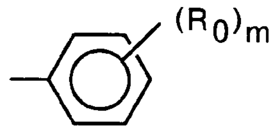

et A symbolise l'un des groupes suivants :
-O-, -COO-, -OCOO-, $-SO_2-$,

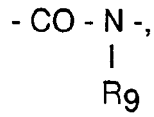

dans ces formules, $R_9$ représente un atome d'hydrogène ou un radical alkyle ayant de 1 à 4 atomes de carbone, un radical cyclohexyle ou phényle.

- deux groupes $R_1$ et $R_2$ et/ou $R_2$ et $R_3$ et/ou $R_3$ et $R_4$ placés sur deux atomes de carbone vicinaux peuvent former ensemble et avec les atomes de carbone qui les portent un carbocycle insaturé ou aromatique ayant de 4 à 7 atomes de carbone et, de préférence, 6 atomes de carbone.

A titre d'exemples de radicaux $R_a$ et $R_b$ convenant bien à la présente invention, on peut citer entre autres, les radicaux alkyle ramifiés ayant au moins 3 atomes de carbone et les radicaux aryle ayant au moins 6 atomes de carbone et plus particulièrement les radicaux tert-butyle, tert-pentyle, tert-hexyle ou phényle éventuellement substitué.

Les fuchsones convenant à la présente invention peuvent être représentées plus précisément par les formules générales (Ia) et/ou (IIa) suivantes :

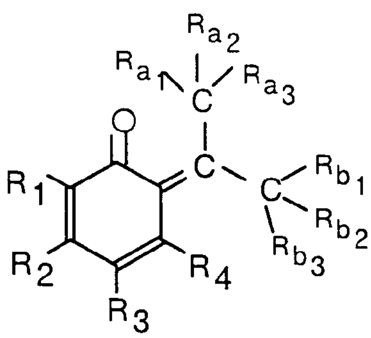

(Ia)

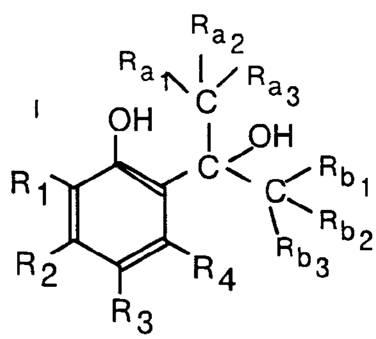

(IIa)

dans lesdites formules (Ia) et (IIa) :

- $R_{a1}$, $R_{a2}$, $R_{a3}$ et $R_{b1}$, $R_{b2}$, $R_{b3}$ identiques ou différents, représentent des radicaux alkyle linéaires ou ramifiés ayant de 1 à 10 atomes de carbone, des radicaux cyclohexyle, phényle ou napthyle éventuellement substitués,
- $R_{a1}$, $R_{a2}$, $R_{a3}$ d'une part et $R_{b1}$, $R_{b2}$, $R_{b3}$, d'autre part peuvent former ensemble et l'atome de carbone qui les porte, un cycle benzénique ou naphtalénique éventuellement substitué.
- $R_1$, $R_2$, $R_3$ et $R_4$, identiques ou différents représentent l'un des groupes suivants :
  . un atome d'hydrogène,
  . un radical alkyle, linéaire ou ramifié ayant de 1 à 4 atomes de carbone,.
  . un radical alkoxy linéaire ou ramifié ayant de 1 à 4 atomes de carbone,
  . un groupe hydroxyle,
  . un atome d'halogène,
  . un groupe $-CF_3$
  . un radical cyclohexyle,
  . un radical phényle,
- les deux groupes $R_1$ et $R_2$ et/ou $R_2$ et $R_3$ et/ou $R_3$ et $R_4$ placés sur deux atomes de carbone vicinaux peuvent former ensemble et avec les atomes de carbone qui les portent un cycle benzénique.

Par "éventuellement substitué", on entend qu'il y a présence de un ou plusieurs substituants dans les radicaux cycliques. Lesdits substituants symbolisés par Y, sont précisés ultérieurement.

Encore plus préférentiellement, on choisit les composés de formule (Ia) et/ou (IIa) dans laquelle l'un des

radicaux $R_1$ $R_2$, $R_3$ et $R_4$ représente un groupe hydroxyle, un radical méthyle ou un radical méthoxy et les 3 autres représentent un atome d'hydrogène.

Parmi toutes les fuchsones répondant à la formule (Ia) et/ou (IIa), on fait appel tout particulièrement aux fuchsones répondant aux formules (Ib) et/ou (IIb) suivantes :

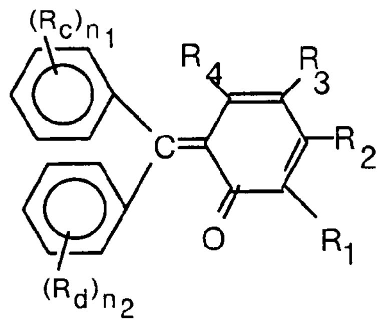

(Ib)

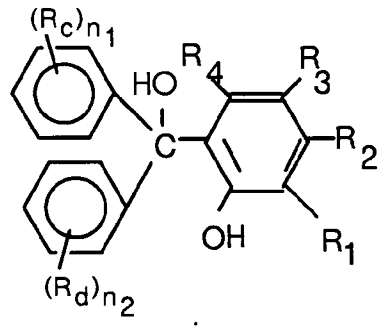

(IIb)

dans lesdites formules (Ib) et (IIb) :

- $R_1$, $R_2$, $R_3$ et $R_4$, identiques ou différents représentent l'un des groupes suivants:
  . un atome d'hydrogène,
  . un radical alkyle linéaire ou ramifié, ayant de 1 à 4 atomes de carbone,.
  . un radical alkoxy linéaire ou ramifié ayant de 1 à 4 atomes de carbone,
  . un groupe hydroxyle,
  . un atome d'halogène,
  . un groupe $-CF_3$
  . un radical cyclohexyle,
  . un radical phényle,
- les deux groupes $R_1$ et $R_2$ et/ou $R_2$ et $R_3$ et/ou $R_3$ et $R_4$ placés sur deux atomes de carbone vicinaux peuvent former ensemble et avec les atomes de carbone qui les portent un cycle benzénique.
- $R_c$ et $R_c$ identiques ou différents représentent un atome d'hydrogène ou un substituant,
- $n_1$, $n_2$ identiques ou différents est un nombre égal à 0, 1, 2 ou 3.

Le substituant désigné Y est choisi de telle sorte qu'il ne réagisse pas dans les conditions d'acidité de l'invention.

Des exemples de substituants convenant bien à l'invention sont les suivants :

- les radicaux alkyle linéaires ou ramifiés ayant de 1 à 4 atomes de carbone,
- le radical phényle,
- les radicaux alkoxy $R_{10}$ - O dans lesquels $R_{10}$ représente un radical alkyle linéaire ou ramifié ayant de 1 à 4 atomes de carbone ou le radical phényle,
- le groupe hydroxyle,
- les atomes d'halogène, de préférence de chlore, de brome ou de fluor.

Comme exemples de fuchsones particulièrement adaptées à l'invention, on peut citer tout particulièrement les fuchsones répondant à la formule générale (Ib) et/ou (IIb) dans laquelle $R_c$ et $R_d$, identiques ou différents représentent un atome d'hydrogène ou un substituant tel que précité ; $R_c$ et $R_d$ étant de préférence en position 4,4' et $n_1$, $n_2$ identiques ou différents sont égaux à 0 ou 1.

On fait appel préférentiellement aux fuchsones répondant à la formule (Ib) et/ou (IIb) dans laquelle $R_c$ et $R_d$ identiques ou différents représentent un atome d'hydrogène un radical méthyle, éthyle, tert-butyle, phényle ; un radical méthoxy ou éthoxy ; un groupe hydroxyle $R_c$ et $R_d$ étant de préférence en position 3,3' ou 4,4'.

Comme exemples spécifiques de fuchsones qui peuvent être utilisées dans le procédé de l'invention, on peut citer plus particulièrement l'o-diphénylfuchsone et/ou son carbinol :

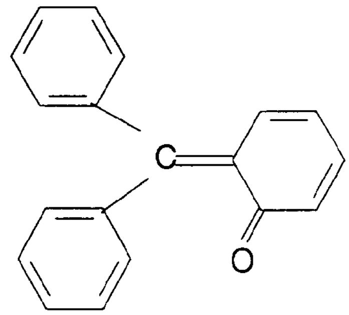

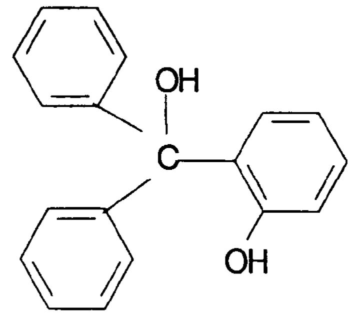

On peut mettre en oeuvre dans le procédé de l'invention, toute fuchsone, quelque soit son mode d'obtention. Ainsi, on peut partir de fuchsones obtenues selon les procédés décrits dans la littérature notamment par M. PISOVA et M. SOUCEK.[Coll. Czech. Chem. Commun. 47 (12) 3318-27] et J.J TALLEY et I.A EVANS. [J. Org. Chem. 1984, 49, 5267-5269].

Comme agents oxydants susceptibles d'être mis en oeuvre dans le procédé de l'invention, on peut citer notamment, le peroxyde d'hydrogène, les peracides tels que l'acide peracétique, les hydroperoxydes tels que l'hydroperoxyde de tertiobutyle, l'hydroperoxyde de cyclohexyle, l'hydroperoxyde de cumyle.

Parmi les agents oxydants précités, on fait appel préférentiellement au peroxyde d'hydrogène.

Le peroxyde d'hydrogène mis en oeuvre selon l'invention peut être sous forme de solution aqueuse ou de solution organique.

Les solutions aqueuses étant commercialement plus facilement disponibles sont utilisées, de préférence.

La concentration de la solution aqueuse de peroxyde d'hydrogène bien que non critique en soi est choisie de façon à introduire le moins d'eau possible dans le milieu réactionnel. On utilise généralement une solution aqueuse de peroxyde d'hydrogène à au moins 20 % en poids de $H_2O_2$ et, de préférence, aux environs de 70 %.

La quantité de peroxyde d'hydrogène est de préférence égale à la quantité stoechiométrique soit 1 mole de $H_2O_2$ pour 1 mole de fuchsone de formule (I) et/ou (II). On peut envisager de mettre en oeuvre un léger excès pouvant atteindre 20 % de la quantité stoechiométrique.

Une variante préférée du procédé de l'invention consiste à faire réagir la fuchsone et l'agent oxydant, en présence d'un catalyseur acide.

Intervient éventuellement dans le procédé de l'invention, un catalyseur acide qui est un acide fort. Par acide fort, on désigne dans la présente invention, un acide présentant un pKa dans l'eau inférieur à - 0,1 et, de préférence, inférieur à - 1,0.

Le pKa est défini comme la constante de dissociation ionique du couple acide/base, lorsque l'eau est utilisée comme solvant.

Parmi les acides répondant à cette définition, il est préférable d'utiliser ceux qui sont stables vis-à-vis de l'oxydation par le peroxyde d'hydrogène.

On peut citer plus particulièrement les oxyacides halogénés ou non tels que l'acide sulfurique, l'acide pyrosulfurique, l'acide perchlorique, les acides halogénosulfoniques tels que l'acide fluorosulfonique, l'acide chlorosulfonique ou l'acide trifluorométhanesulfonique, l'acide méthanesulfonique, l'acide éthanesulfonique, l'acide éthanedisulfonique, l'acide benzènesulfonique, les acides benzènedisulfoniques, les acides toluènesulfoniques, les acides naphtalènesulfoniques et les acides naphtalènedisulfoniques.

Parmi ces acides, on utilisera de préférence l'acide perchlorique, l'acide trifluorométhanesulfonique, l'acide paratoluènesulfonique, l'acide chlorosulfonique, l'acide fluorosulfonique, l'acide méthanesulfonique.

On choisit, tout particulièrement, l'acide perchlorique, l'acide trifluorométhanesulfonique ou l'acide méthanesulfonique.

La quantité d'acide exprimée par le rapport du nombre d'équivalents de protons au nombre de moles de peroxyde d'hydrogène peut varier entre environ $1.10^{-4}$ et environ 1,0.

Une variante préférée du procédé de l'invention consiste à choisir un rapport $H^+/H_2O_2$ compris entre $1.10^{-3}$ et 0,1.

La réaction est avantageusement conduite en phase liquide. Ainsi, on commence par solubiliser la fuchsone dans un solvant organique qui peut être le composé aromatique dihyroxylé à obtenir et/ou tout solvant organique protique ou aprotique, polaire ou apolaire dans la mesure où il convient à la présente invention.

Plusieurs impératifs président au choix du solvant.

Le solvant organique doit être stable dans les conditions de l'invention. Sont exclus de la présente invention, les solvants qui ne sont pas stables dans le milieu réactionnel et qui se dégradent par oxydation.

Le solvant organique doit, de préférence, solubiliser à la fois l'agent oxydant et la fuchsone présente.

De plus, il est souhaitable que le solvant organique ne soit pas trop basique c'est-à-dire que sa basicité soit telle qu'il possède un "nombre donneur" inférieur à 25. Pour apprécier la basicité d'un solvant, on se réfère au "nombre donneur". On rappelera que le "nombre donneur" ou "donor number" désigné de manière abrégée DN, donne une indication sur le caractère nucléophile du solvant et révèle son aptitude à donner son doublet.

Dans l'ouvrage de Christian REINHARDT, [Solvents and Solvent Effects in Organic Chemistry - VCH p.19 (1988)], on trouve la définition du "donor number" qui est défini comme le négatif (-ΔH) de l'enthalpie (Kcal/mol) de l'interaction entre le solvant et le pentachlorure d'antimoine, dans une solution diluée de dichloroéthane.

Comme exemples de solvants organiques susceptibles d'être mis en oeuvre dans le procédé de l'invention, on peut citer plus particulièrement :

- le phénol,
- les nitriles alphatiques ou aromatiques comme l'acétonitrile, le propionitrile, le butanenitrile, l'isobutanenitrile, le benzonitrile, le cyanure de benzyle,
- les éther-oxydes aliphatiques, cycloaliphatiques ou aromatiques et, plus particulièrement, l'oxyde de diéthyle, l'oxyde de dipropyle, l'oxyde de diisopropyle, l'oxyde de dibutyle, le méthyltertiobutyléther, l'oxyde de dipentyle, l'oxyde de diisopentyle, le diméthyléther de l'éthylèneglycol (ou diméthoxy-1,2 éthane), le diméthyléther du diéthylèneglycol (ou diméthoxy-1,5 oxa-3 pentane), le dioxane, le tétrahydrofuranne,
- les hydrocarbures aliphatiques chlorés, par exemple, le dichlorométhane, le tétrachlorométhane, le dichloroéthane,
- les alcools tels que le méthanol, l'éthanol, l'isopropanol, le tertiobutanol.

On peut également utiliser un mélange de solvants et, en particulier un mélange du composé phénolique à obtenir et d'un solvant organique.

La quantité de solvant organique mise en oeuvre est fonction de la solubilité de la fuchsone dans ledit solvant organique.

La concentration de la fuchsone dans le solvant organique peut varier largement. Elle est avantageusement comprise entre 0,1 et 2 moles/litre.

Conformément au procédé de l'invention, on fait réagir la fuchsone et l'agent oxydant, éventuellement en présence d'un catalyseur acide à une température qui peut être comprise entre 20°C et 150°C.

Une variante préférée du procédé de l'invention consiste à choisir la température entre 30°C et 80°C.

La réaction est conduite avantageusement sous pression atmosphérique.

D'un point de vue pratique, le procédé selon l'invention est simple à mettre en oeuvre de façon continue ou discontinue.

Les différents réactifs peuvent être introduits dans n'importe quel ordre dans la mesure où l'agent oxydant est introduit en dernier. D'une manière préférée, on choisit l'ordre des réactifs suivants : on introduit la fuchsone de formule (I) et/ou (II), le solvant réactionnel et le catalyseur acide.

On porte le milieu réactionnel à la température désirée puis, l'on ajoute l'agent oxydant de préférence une solution de peroxyde d'hydrogène, de manière progressive.

En fin de réaction, les produits d'hydroxylation sont séparés selon les moyens usuels, notamment, par distillation.

Le procédé de l'invention permet d'obtenir sélectivement des composés aromatiques o-dihydroxylés.

On donne ci-après des exemples de réalisation de l'invention.

Les exemples qui suivent, illustrent l'invention sans toutefois la limiter.

Dans les exemples, les abréviations suivantes signifient :

$$RR_{PC/FUCHSONE} = \frac{\text{nombre de moles de pyrocatéchine formées}}{\text{nombre de moles de fuchsone introduites}} \%$$

## EXEMPLES

### Exemple 1

La synthèse de l'o-diphénylfuchsone qui est mise en oeuvre dans cet exemple, est effectuée d'une manière connue, selon les travaux de M. PISOVA et M. SOUCEK. [Coll. Czech. Chem. Commun. 47 (12) 3318-27]. Elle répond à la formule :

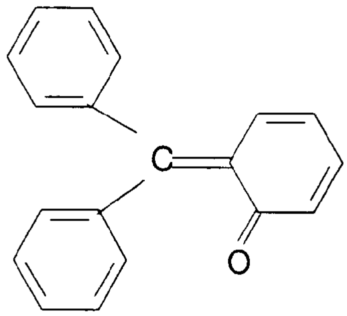

On effectue ensuite la préparation de la pyrocatéchine, selon le procédé de l'invention.

Dans un ballon de 25 ml muni d'une agitation par barreau aimanté, on charge :

- 0,774 g (3 mmol) d'o-diphénylfuchsone,
- 8,3 ml d'acétonitrile,
- 0,113 ml (3mmol) de peroxyde d'hydrogène sous forme d'une solution aqueuse à 70% en poids,
- et 2 gouttes d'acide perchlorique à 70% en poids.

La température s'élève légèrement, puis on chauffe à 30°C durant 1 heure.

Les produits de la réaction sont dosés directement par chromatographie liquide haute performance.

Dans le milieu réactionnel, on dose:

- 2,40 mmol de pyrocatéchine RR = 80 %
- 2,40 mmol de benzophénone RR = 80 %

Exemple 2

Dans cet exemple, on met en oeuvre l'hydroxy-2 triphénylcarbinol qui est préparé selon J.J TALLEY et I.A EVANS. [J. Org. Chem. 1984, 49, 5267-5269].

Dans un réacteur d'un litre en verre muni d'une agitation centrale, d'une ampoule de coulée de 250 ml, d'un réfrigérant droit, d'une prise de température, l'ensemble étant maintenu sous atmosphère d'argon, on coule 22,5 g (0,13 mol) d'orthobromophénol en solution dans 100 ml d'éther éthylique anhydre sur un mélange de 164 ml (0,26 mol) de n-butyllithium en solution hexanique et de 200 ml d'éther. Pendant la durée de la coulée (45 mn), on maintient la température du milieu vers 0°C, puis on laisse remonter naturellement à la température ambiante. La durée totale de la réaction est de 2 heures 45 mn.

La condensation de la benzophénone sur l'organolithien s'effectue par coulée en 30 mn d'une solution éthérée de 23,7 g (0,13 mol) de benzophénone à une température voisine de - 70°C, puis on remonte la température à l'ambiante. La durée totale est de 1 heure.

On hydrolyse ensuite le milieu réactionnel en coulant lentement une solution aqueuse de chlorure d'ammonium, en contrôlant l'exothermie, à une température voisine de 0°C. La phase étherée séparée de la couche aqueuse, est lavée à l'eau, séchée sur sulfate de magnésium et évaporée sous vide.

Le résidu obtenu est recristallisé de chaud à froid dans 350 ml de cyclohexane additionné de 0,2 g de noir 3S.

On obtient après séchage, 17,9 g de solide blanc de formule :

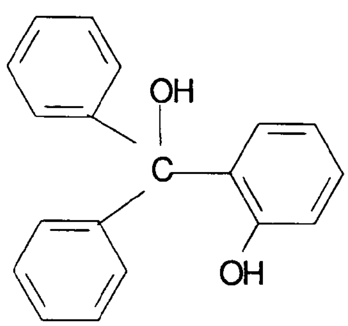

Il a un point de fusion de 152°C. Son spectre RMN 1H, 13C est conforme.

On effectue ensuite la préparation de la pyrocatéchine à partir de l'hydroxy-2 triphénylcarbinol précédemment obtenu.

Dans un ballon de 25 ml muni d'une agitation par barreau aimanté, on charge 0,828 g (3 mmol) de l'hydroxy-2 triphénylcarbinol, 8,3 ml d'acétonitrile, 0,113 ml d'eau oxygènée à 70% (3 mmol) et 2 gouttes d'acide perchlorique à 70%.

La température s'élève légèrement, puis on chauffe à 70°C durant 1 heure.

Les produits de la réaction sont dosés directement par chromatographie en phase gazeuse et par chromatographie liquide haute performance.

Dans le milieu réactionnel, on dose :

- 2,44 mmol de pyrocatéchine RR = 81,3 %
- 2,40 mmol de benzophénone RR = 80 %

**Revendications**

**1 -** Procédé de préparation d'un composé aromatique o-dihydroxylé caractérisé par le fait qu'il consiste à faire réagir un agent oxydant, avec une o-fuchsone.

**2 -** Procédé selon la revendication 1 caractérisé par le fait qu'il consiste à faire réagir un agent oxydant avec une fuchsone répondant à la formule générale (I) et/ou (II) :

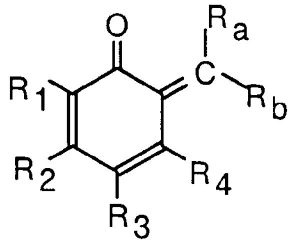

(I)

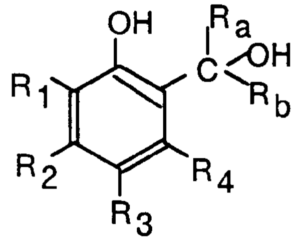

(II)

. dans lesdites formules (I) et (II), les différents symboles $R_1$, $R_2$, $R_3$, $R_4$, $R_a$ et $R_b$ ont la signification suivante :

- $R_a$ et $R_b$, identiques ou différents sont des radicaux hydrocarbonés ayant chacun de 3 à 30 atomes de carbone ; les atomes de carbone de chaque radical $R_a$ et $R_b$ en position $\alpha$ par rapport à l'atome de carbone qui les porte, étant des carbones tertiaires,
- $R_1$, $R_2$, $R_3$ et $R_4$, identiques ou différents représentent un atome d'hydrogène ou un substituant quelconque,
- deux groupes $R_1$ et $R_2$ et/ou $R_2$ et $R_3$ et/ou $R_3$ et $R_4$ placés sur deux atomes de carbone vicinaux peuvent former ensemble et avec les atomes de carbone qui les portent un cycle.

**3 -** Procédé selon l'une des revendications 1 et 2 caractérisé par le fait que la fuchsone est une fuchsone répondant à la formule générale (I) et/ou (II) dans laquelle :

- $R_1$, $R_2$, $R_3$ et $R_4$, identiques ou différents représentent $R_0$, l'un des groupes suivants :
  . un atome d'hydrogène,
  . un radical alkyle, linéaire ou ramifié, ayant de 1 à 6 atomes de carbone, de préférence de 1 à 4 atomes de carbone, tel que méthyle, éthyle, propyle, isopropyle, butyle, sec-butyle, tert-butyle,
  . un radical alcényle linéaire ou ramifié ayant de 2 à 6 atomes de carbone, de préférence, de 2 à 4 atomes de carbone, tel que vinyle, allyle,
  . un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy,
  . un groupe acyle ayant de 2 à 6 atomes de carbone,
  . un radical de formule :

$$-R_0\text{-}OH$$
$$-R_5\text{-}COOR_6$$
$$-R_0\text{-}X$$
$$-R_0\text{-}CF_3$$

dans lesdites formules, $R_0$ représente un lien valentiel ou un radical hydrocarboné divalent, linéaire ou ramifié, saturé ou insaturé, ayant de 1 à 6 atomes de carbone tel que, par exemple, méthylène, éthylène, propylène, isopropylène, isopropylidène ; $R_6$ représente un atome d'hydrogène ou un radical alkyle linéaire ou ramifié ayant de 1 à 6 atomes de carbone ; X symbolise un atome d'halogène, de préférence un atome de chlore, de brome ou de fluor.

- $R_1$, $R_2$, $R_3$ et $R_4$, identiques ou différents représentent $R_7$, l'un des radicaux plus complexes suivants :

. un radical carbocyclique saturé ou insaturé ayant de 4 à 7 atomes de carbone, de préférence, un radical cyclohexyle,

. un radical de formule

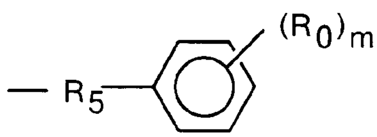

dans lequel $R_5$ représente un lien valentiel ou un radical hydrocarboné divalent, linéaire ou ramifié, saturé ou insaturé ayant de 1 à 6 atomes de carbone tel que par exemple, méthylène, éthylène, propylène, isopropylène, isopropylidène et $R_0$ ayant la signification donnée précédemment et m est un nombre entier de 0 à 4,

. un radical - $R_5$ - A - $R_0$ dans lequel $R_5$ a la signification donnée précédemment, $R_8$ représente un radical alkyle linéaire ou ramifié ayant de 1 à 6 atomes de carbone ou un radical

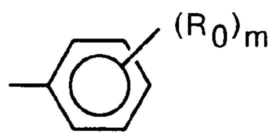

et A symbolise l'un des groupes suivants :
-O-, -COO-, -OCOO-, - $SO_2$-

- CO - N -,
I
$R_9$

dans ces formules, $R_9$ représente un atome d'hydrogène ou un radical alkyle ayant de 1 à 4 atomes de carbone, un radical cyclohexyle ou phényle.

- deux groupes $R_1$ et $R_2$ et/ou $R_2$ et $R_3$ et/ou $R_3$ et $R_4$ placés sur deux atomes de carbone vicinaux peuvent former ensemble et avec les atomes de carbone qui les portent un carbocycle insaturé ou aromatique ayant de 4 à 7 atomes de carbone et, de préférence, 6 atomes de carbone.

**4 -** Procédé selon l'une des revendications 1 à 3 caractérisé par le fait que la fuchsone est une fuchsone répondant à la formule générale (I) et/ou (II) dans laquelle $R_a$ et $R_b$ représentent un radical alkyle ramifié ayant au moins 3 atomes de carbone ou un radical aryle ayant au moins 6 atomes de carbone, de préférence un radical tert-butyle, tert-pentyle, tert-hexyle ou phényle éventuellement substitué.

**5 -** Procédé selon l'une des revendications 1 à 4 caractérisé par le fait que la fuchsone est une fuchsone répondant à la formule générale (Ia) et/ou (IIa) suivante :

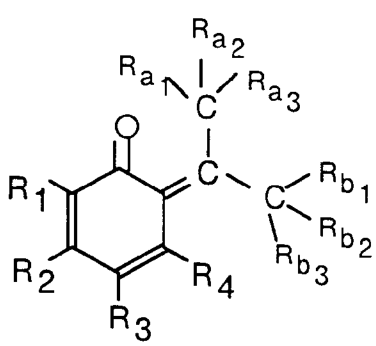

(Ia)

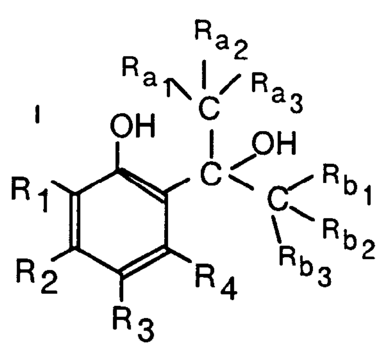

(IIa)

dans lesdites formules (Ia) et (IIa) :

- $R_{a1}$, $R_{a2}$, $R_{a3}$ et $R_{b1}$, $R_{b2}$, $R_{b3}$ identiques ou différents, représentent des radicaux alkyle linéaires ou ramifiés ayant de 1 à 10 atomes de carbone, des radicaux cyclohexyle, phényle ou napthyle éventuelle-

ment substitués,

- $R_{a1}$, $R_{a2}$, $R_{a3}$ d'une part et $R_{b1}$, $R_{b2}$, $R_{b3}$, d'autre part peuvent former ensemble et l'atome de carbone qui les porte, un cycle benzénique ou naphtalénique éventuellement substitué.
- $R_1$, $R_2$, $R_3$ et $R_4$, identiques ou différents représentent l'un des groupes suivants :
  . un atome d'hydrogène,
  . un radical alkyle, linéaire ou ramifié ayant de 1 à 4 atomes de carbone,.
  . un radical alkoxy linéaire ou ramifié ayant de 1 à 4 atomes de carbone,
  . un groupe hydroxyle,
  . un atome d'halogène,
  . un groupe $-CF_3$
  . un radical cyclohexyle,
  . un radical phényle,
- les deux groupes $R_1$ et $R_2$ et/ou $R_2$ et $R_3$ et/ou $R_3$ et $R_4$ placés sur deux atomes de carbone vicinaux peuvent former ensemble et avec les atomes de carbone qui les portent un cycle benzénique.

**6 -** Procédé selon l'une des revendications 1 à 5 caractérisé par le fait que la fuchsone est une fuchsone répondant à la formule générale (Ia) et/ou (IIa) dans laquelle l'un des radicaux $R_1$ $R_2$, $R_3$ et $R_4$ représente un groupe hydroxyle, un radical méthyle ou un radical méthoxy et les 3 autres représentent un atome d'hydrogène.

**7 -** Procédé selon l'une des revendications 1 à 6 caractérisé par le fait que la fuchsone est une fuchsone répondant à la formule (Ib) et/ou (IIb) suivante :

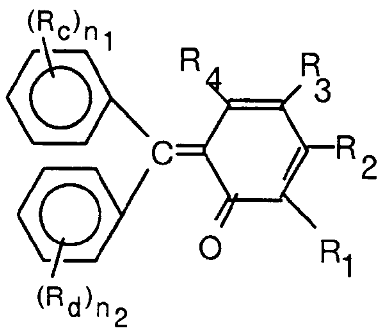

(Ib)

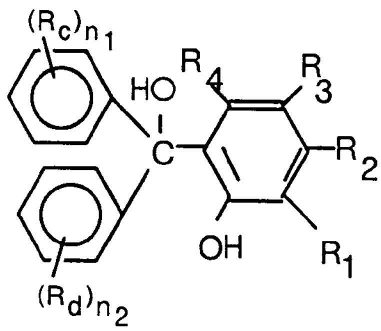

(IIb)

dans lesdites formules (Ib) et (IIb) :

- $R_1$, $R_2$, $R_3$ et $R_4$, identiques ou différents représentent l'un des groupes suivants :
  . un atome d'hydrogène,
  . un radical alkyle, linéaire ou ramifié, ayant de 1 à 4 atomes de carbone,.
  . un radical alkoxy linéaire ou ramifié ayant de 1 à 4 atomes de carbone,
  . un groupe hydroxyle,
  . un atome d'halogène,
  . un groupe $-CF_3$
  . un radical cyclohexyle,
  . un radical phényle,
- les deux groupes $R_1$ et $R_2$ et/ou $R_2$ et $R_3$ et/ou $R_3$ et $R_4$ placés sur deux atomes de carbone vicinaux peuvent former ensemble et avec les atomes de carbone qui les portent un cycle benzénique.
- $R_c$ et $R_c$ identiques ou différents représentent un atome d'hydrogène ou un substituant,
- $n_1$, $n_2$ identiques ou différents est un nombre égal à 0, 1, 2 ou 3.

**8 -** Procédé selon l'une des revendications 1 à 7 caractérisé par le fait que la fuchsone est une fuchsone répondant à la formule (Ib) et/ou (IIb) dans laquelle $R_c$ et $R_d$, identiques ou différents représentent :

- des radicaux alkyle linéaires ou ramifiés ayant de 1 à 4 atomes de carbone,
- un radical phényle,
- des radicaux alkoxy $R_{10}$ - O dans lesquels $R_{10}$ représente un radical alkyle linéaire ou ramifié ayant de 1 à 4 atomes de carbone ou le radical phényle,
- un groupe hydroxyle,
- un atome d'halogène.

**9 -** Procédé selon l'une des revendications 1 à 8 caractérisé par le fait que la fuchsone est une fuchsone répondant à la formule (Ib) et/ou (IIb) dans laquelle $R_c$ et $R_c$, identiques ou différents représentent un atome d'hydrogène ou un substituant tel que défini dans la revendication 8, de préférence en position 4,4' et $n_1$, $n_2$ identiques ou différents sont égaux à 0 ou 1.

**10 -** Procédé selon l'une des revendications 1 à 9 caractérisé par le fait que la fuchsone est une fuchsone répondant à la formule (Ib) et/ou (IIb) dans laquelle $R_c$ et $R_c$, identiques ou différents représentent un atome d'hydrogène ; un radical méthyle, éthyle, tert-butyle, phényle ; un radical méthoxy ou éthoxy ; un groupe hydroxyle, de préférence en position 3,3' ou 4,4'.

**11 -** Procédé selon l'une des revendications 1 à 10 caractérisé par le fait que la fuchsone répondant à la formule générale (Ib) et/ou (IIb) est : l'o-diphénylfuchsone, sous forme anhydre, hydratée ou en mélange.

**12 -** Procédé selon l'une des revendications 1 à 11 caractérisé par le fait que l'agent oxydant est choisi parmi le peroxyde d'hydrogène ; les peracides de préférence, l'acide peracétique ; les hydroperoxydes de préférence l'hydroperoxyde de tertiobutyle, l'hydroperoxyde de cyclohexyle, l'hydroperoxyde de cumyle.

**13 -** Procédé selon la revendication 12 caractérisé par le fait que l'agent oxydant est une solution aqueuse de peroxyde d'hydrogène.

**14 -** Procédé selon l'une des revendications 1 à 13 caractérisé par le fait que la quantité de peroxyde d'hydrogène est de préférence égale à la quantité stoechiométrique soit 1 mole de $H_2O_2$ pour 1 mole de fuchsone de formule (I) et/ou (II).

**15 -** Procédé selon l'une des revendications 1 à 14 caractérisé par le fait que l'on fait réagir la fuchsone et l'agent oxydant, en présence d'un catalyseur acide qui est un acide fort.

**16 -** Procédé selon la revendication 15 caractérisé par le fait que l'acide fort est un oxyacide halogéné ou non, de préférence, l'acide sulfurique, l'acide pyrosulfurique, l'acide perchlorique, les acides halogénosulfoniques, de préférence, l'acide fluorosulfonique, l'acide chlorosulfonique ou l'acide trifluorométhanesulfonique, l'acide méthanesulfonique, l'acide éthanesulfonique, l'acide éthanedisulfonique, l'acide benzènesulfonique, les acides benzènedisulfoniques, les acides toluènesulfoniques, les acides naphtalènesulfoniques et les acides naphtalènedisulfoniques.

**17 -** Procédé selon la revendication 16 caractérisé par le fait que l'acide fort est l'acide perchlorique, l'acide trifluorométhanesulfonique ou l'acide méthanesulfonique.

**18 -** Procédé selon l'une des revendications 1 à 17 caractérisé par le fait que la quantité d'acide exprimée par le rapport du nombre d'équivalents de protons au nombre de moles de peroxyde d'hydrogène varie entre environ $1.10^{-4}$ et environ 1,0, de préférence, entre $1.10^{-3}$ et 0,1.

**19 -** Procédé selon l'une des revendications 1 à 18 caractérisé par le fait que la réaction est conduite en présence d'un solvant organique, de préférence choisi parmi :

- le phénol,
- les nitriles alphatiques ou aromatiques, de préférence, l'acétonitrile, le propionitrile, le butanenitrile, l'isobutanenitrile, le benzonitrile, le cyanure de benzyle,
- les éther-oxydes aliphatiques, cycloaliphatiques ou aromatiques et, de préférence, l'oxyde de diéthyle, l'oxyde de dipropyle, l'oxyde de diisopropyle, l'oxyde de dibutyle, le méthyltertiobutyléther, l'oxyde de dipentyle, l'oxyde de diisopentyle, le diméthyléther de l'éthylèneglycol (ou diméthoxy-1,2 éthane), le diméthyléther du diéthylèneglycol (ou diméthoxy-1,5 oxa-3 pentane), le dioxane, le tétrahydrofuranne,
- les hydrocarbures aliphatiques chlorés, de préférence, le dichlorométhane, le tétrachlorométhane, le dichloroéthane,
- les alcools, de préférence, le méthanol, l'éthanol, l'isopropanol, le tertiobutanol.

**20 -** Procédé selon l'une des revendications 1 à 19 caractérisé par le fait que la température de la réaction est comprise entre 20°C et 150°C, de préférence entre 30°C et 80°C.

Office européen des brevets

# RAPPORT DE RECHERCHE EUROPEENNE

Numero de la demande
EP 94 40 0019

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.5) |
|---|---|---|---|
| D,A | FR-A-2 071 464 (SOCIÉTÉ DES USINES CHIMIQUES RHONE - POULENC)<br>* le document en entier * | 1-20 | C07C37/60<br>C07C39/08 |
| D,A | CHEMICAL ABSTRACTS, vol. 098, no. 21, 23 Mai 1983, Columbus, Ohio, US; abstract no. 178889, PISOVA M ET AL 'Quinone methides and fuchsones. XXVII. Oxidative rearrangement of o-fuchsone to 2,2-diphenyl-1,3-benzodioxole'<br>* abrégé *<br>& COLLECT. CZECH. CHEM. COMMUN. (CCCCAK,0366547X);82; VOL.47 (12); PP.3318-27 CZECH. ACAD. SCI.;INST. ORG. CHEM. BIOCHEM.; PRAGUE; 166 10/6; CZECH. (CS) | 1 | |
| A | US-A-4 078 006 (S. UMEMURA ET AL.)<br>* revendications * | 1 | |
| A | FR-A-2 336 364 (RHONE-POULENC INDUSTRIES)<br>* revendications; exemples 8,9 * | 1 | |

| DOMAINES TECHNIQUES RECHERCHES (Int.Cl.5) |
|---|
| C07C |

Le présent rapport a été établi pour toutes les revendications

| Lieu de la recherche | Date d'achèvement de la recherche | Examinateur |
|---|---|---|
| LA HAYE | 27 Avril 1994 | Bonnevalle, E |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)